# EUROPÄISCHE PATENTSCHRIFT

(11) **EP 4 171 450 B1**
(45) Veröffentlichungstag und Bekanntmachung des Hinweises auf die Patenterteilung: **20.08.2025**
(21) Anmeldenummer: 21736962.8
(22) Anmeldetag: 11.06.2021
(51) Int. Cl.: A61F 2/962, A61F 2/94, A61F 11/20

(54) **APPLIKATOR UND STENT FÜR EINE EUSTACHISCHE RÖHRE**
APPLICATOR AND STENT FOR A EUSTACHIAN TUBE
APPLICATEUR ET STENT POUR TROMPE D'EUSTACHE

(30) Priorität: 25.06.2020 DE 102020116795
(43) Veröffentlichungstag der Anmeldung: 03.05.2023
(73) Patentinhaber: Bess Pro GmbH, 14167 Berlin (DE); Medizinische Hochschule Hannover, 30625 Hannover (DE)
(72) Erfinder: KRÜGER, Philipp, 12247 Berlin (DE); LANGE, Sven Karsten, 13591 Berlin (DE); LENARZ, Thomas, 30559 Hannover (DE); PAASCHE, Gerrit, 39615 Werben / OT Berge (DE); SCHUON, Robert, 48155 Münster (DE)
(74) Vertreter: Griepenstroh, Jörg
(86) Internationale Anmeldenummer: PCT/DE2021/100501
(87) Internationale Veröffentlichungsnummer: WO 2021/259418

(56) Entgegenhaltungen:
- EP-B1- 2 532 300
- CN-A- 109 998 747
- US-A1- 2007 043 381
- US-A1- 2018 001 065
- US-A1- 2019 060 125

## Beschreibung

Die Erfindung betrifft ein System umfassend einen Applikator und einen Stent für eine eustachische Röhre.

Viele Menschen leiden an chronischen Tubenbelüftungsstörungen. Bei gestörter Ventilfunktion der Ohrtrompete (Tuba Eustachii - Eustachische Röhre) ist der Druckausgleich zwischen Mittelohr und Umgebung nicht mehr hinreichend möglich. Das führt - neben Missempfindungen bis hin zu stärksten Ohrschmerzen - zu einer Schallleitungs- bzw. kombinierten Schwerhörigkeit, vor allem durch entzündliche Prozesse.

Das Valsalva-Manöver, bei welchem der Patient versucht, kräftig auszuatmen, während er sich für etwa 10 Sekunden die Nase zuhält und den Mund verschließt, als therapeutisches Verfahren repetitiv durchgeführt bzw. apparative Hilfen, wie Nasenballons, die durch die Nase aufgeblasen werden, gewähren häufig keine dauerhaft ausreichende Mittelohrbelüftung und -drainage, sodass eine Paracentese bzw. Paukendrainage durch einen kleinen operativen Eingriff zur hinreichenden Mittelohrbelüftung notwendig sind. Dabei greift die Therapie gerade an einer der empfindlichsten Stellen des Mittelohres an: dem Trommelfell. Dieser wesentliche Teil der Schallleitungskette ist bei chronisch erkranktem Mittelohr meist atroph und narbig. Paukendrainagen stellen heute die beste verfügbare Therapie dar, jedoch ist bei der Paukendrainage die protektive Funktion der Tube ausgehebelt.

Eine interventionelle Methode bzw. Therapie der Tube selbst ist inzwischen als Ballondilatation der Tuba Eustachii (BET) bekannt. Analog einer kardiovaskulären Ballondilatation bei koronarer Herzerkrankung wird hier der knorpelige Anteil der Ohrtrompete kurzfristig mit einem Ballonkatheter geweitet. Dieses Verfahren hat sich als nahezu nebenwirkungsfrei herausgestellt. Allerdings kommt die Freiheit von Nebenwirkungen auch mit einer nicht zweifelsfrei nachgewiesenen Wirkweise einher. Allem Anschein nach kann eine einmalige Aufdehnung der ohnehin schlaffen Gewebestrukturen nur einen kurzfristigen Verbesserungseffekt erzielen. Bei einer chronischen Tubenfunktionsstörung sind in der Regel die Muskelgruppen, die für ein regelmäßiges Öffnen und Schließen der Tube sorgen, nicht oder nur schwach funktionsfähig.

Die US 2019/060125 A1 offenbart einen Applikator zum Platzieren eines Stents in einer eustachischen Röhre, wobei der Applikator ein proximales Ende zur Handhabung des Applikators oder distales Ende zur Aufnahme eines in der eustachischen Röhre platzierbaren Stents besitzt. Das distale Ende weist ein Innenteil und einen Außentubus auf, der das Innenteil im radialen Abstand umgibt und der einen Ringspalt zur Aufnahme des Stents begrenzt. Der Stent in dem Ringspalt besteht aus Nitinol und ist selbstexpandierend. Das Innenteil ist zumindest bereichsweise plastisch verformbar ausgebildet zur Anpassung an einen patientenabhängigen Zugangswinkel der eustachischen Röhre.

Hiervon ausgehend liegt der Erfindung die Aufgabe zu Grunde, eine Möglichkeit aufzuzeigen, chronische Tubenbelüftungsstörungen nachhaltig zu behandeln.

Diese Aufgabe wird durch ein System mit den Merkmalen des Patentanspruchs 1 gelöst.

Die Unteransprüche betreffen zweckmäßige Weiterbildungen der Erfindung.

Der erfindungsgemäße Applikator zum Platzieren eines Stents in einer eustachischen Röhre besitzt ein proximales Ende zur Handhabung des Applikators und ein distales Ende zur Aufnahme eines in einer eustachischen Röhre platzierbaren Stents. Das Besondere des Applikators ist das distale Ende. Es besitzt ein Innenteil und einen Außentubus. Der Außentubus umgibt im radialen Abstand das Innenteil, sodass ein Ringspalt zur Aufnahme eines Stents gebildet wird. Der Außentubus kann zurückgezogen werden, um einen im Ringspalt angeordneten Stent zur Platzierung frei zu geben. Das Innenteil des distalen Endes des Applikators ist zumindest bereichsweise plastisch verformbar ausgebildet, zur Anpassung an einen patientenabhängigen Zugangswinkel zur eustachischen Röhre.

Starre Applikatoren sind nicht oder nicht hinreichend geeignet, die anatomischen Gegebenheiten der Tube zu berücksichtigen. Bei einer Ballondilatation werden endseitig flexible Endoskope verwendet. Die Erfindung sieht jedoch nicht vor, eine einmalige Aufdehnung der ohnehin schlaffen Gewebestruktur vorzunehmen, sondern vielmehr, einen Applikator aufzuzeigen, der dazu ausgebildet ist, einen elastischen Stent in die Tube einzubringen, um für eine nachhaltige Verbesserung zu sorgen. Ein Stent unterstützt die schwachen Muskeln, die für das aktive Öffnen der eustachischen Röhre erforderlich sind durch eine erhöhte Vorspannung und/oder er erleichtert das passive Öffnen der Tube bei großen Druckunterschieden. In beiden Fällen hat die Applikation eines Stents zum Verbleib in der Tube erhebliche Vorteile gegenüber der einmaligen oder wiederholt notwendigen Ballondilatation.

Die Erfindung sieht hierfür einen besonderen Applikator vor. Der Applikator verfügt über einen plastisch verformbaren Anteil, welcher die Anpassung des Applikators an die vorliegende menschliche Anatomie, insbesondere an einen patientenabhängigen Tubenwinkel ermöglicht. Nach dem Einführen des Applikators in die Tube kann der bereits im Applikator geladene Stent durch Betätigen einer Freisetzeinheit an den gewünschten Ort platziert werden.

Der Applikator besitzt an seinem distalen Ende einen biegbaren Bereich, der nicht in eine bestimmte Richtung vorgeformt ist, sondern mittels Handkraft verformbar ist. Das Verformen bzw. Biegen in die gewünschte Richtung und in dem gewünschten Umfang kann vor dem Eingriff durch den Anwender entsprechend der Anatomie des Patienten erfolgen. Es ist lediglich Handkraft erforderlich. Werkzeuge werden nicht benötigt. Das Biegen ist dadurch besonders schonend.

Das verformbare Innenteil weist bevorzugt eine Länge in einem Bereich von 20 mm bis 40 mm auf. Der zu applizierende Stent weist vorzugsweise eine Länge von 8 bis 40 mm auf. Versuche haben gezeigt, dass der zu applizierende Stent im Normalfall im expandierten Zustand einen Außendurchmesser von ca. 5 mm nicht überschreiten muss.

Das Innenteil ist in Verlängerung eines Schaftes des Applikators angeordnet. Der Übergang zwischen dem Schaft und dem Innenteil ist fließend, bzw. tangential gerundet oder auch bogenförmig. Der Schaft ist bei normaler Anwendung elastisch verformbar. Das distale Ende des verformbaren Innenteils kann nach dem Verformen eine Orientierung aufweisen, die deutlich von der Längsachse des Innenteils an seinem proximalen Ende, d.h. von der Längsachse des Schaftes abweicht. Die Abweichung, d.h. der eingeschlossene Winkel zwischen dem distalen Ende und dem proximalen Ende des Innenteils, kann bis zu 70° betragen.

In vorteilhafter Weiterbildung der Erfindung ist der Außentubus zumindest im Bereich der Länge des verformbaren Innenteils formstabil. Der Außentubus ist ebenso wie das Innenteil mittels Handkraft verformbar. Vorzugsweise sind beide Bauteile gemeinsam mittels Handkraft verformbar. Die Formstabilität wird dadurch erreicht, dass auch der Außentubus aus einem Werkstoff besteht, der plastisch verformbar ist. Der Außentubus kann zumindest im vorderen, zu biegenden Abschnitt aus einem anderen Material bestehen als in seinen übrigen Bereichen. Der Außentubus kann aus einem anderen Material als das Innenteil und/oder der Schaft bestehen. Dort wo der Außentubus nicht plastisch verformbar sein muss, ist er es bevorzugt auch nicht. Er ist dort bevorzugt elastisch verformbar.

Die plastische Verformbarkeit von Außentubus und Innenteil stellt sicher, dass der zwischen der Innenseite des Außentubus und der Außenseite des Innenteils angeordnete Ringspalt nicht ungleichmäßig eingeengt wird, was das Freigeben des Stents behindern könnte. Vorzugsweise behält der Ringspalt einen im Wesentlichen gleichbleibenden Durchmesser, sodass die Applikation nicht durch eine Klemmung zwischen Außentubus und Innenteil behindert wird.

Für eine atraumatische Anwendung besitzt der Applikator an seinem äußersten distalen Ende eine vorzugsweise gerundete Spitze. Diese Spitze soll einen Durchmesser von 1,9 bis 2,6 mm aufweisen. Der Stent selber soll vorzugsweise auf einen Durchmesser von 1 bis 2,5 mm gecrimpt werden, insbesondere in Anpassung an den Durchmesser der gerundeten Spitze.

Das plastisch verformbare Innenteil besteht insbesondere aus einem Metall, insbesondere aus einem Metallrohr, mit einer Verdickung in Form einer Olive an der Spitze. Auf den sich an die Olive anschließenden und im Durchmesser reduzierten Bereich des Innenteils wird der Stent gecrimpt und mit dem Außentubus des Applikators gesichert. Vor dem Eingriff kann der Anwender den besagten, im Durchmesser reduzierten Bereich des Innenteils biegen.

Der Schaft des Applikators besitzt vorzugsweise einen Durchmesser von 1,8 bis 2,3 mm. Er kann zusätzlich einen Arbeitskanal aufweisen mit einem Durchmesser kleiner oder gleich 0,8 mm. Der Arbeitskanal kann dafür benutzt werden, eine bewegliche Lichtleitfaser in dem Arbeitskanal anzuordnen oder in den Arbeitskanal einzuschieben. Eine Lichtleitfaser dient zur Lagekontrolle des distalen Endes des Applikators bzw. des Stents vor dem Freisetzen des Stents. Die Lichtleitfaser kann im Sinne einer Diaphanoskopie verwendet werden. Die Lichtleitfaser kann auch vor dem Freisetzen des Stents vorgeschoben werden. Die Lichtleitfaser wird mit einer externen Lichtleitquelle verbunden. Eine Lichtleitfaser kann fest in den Applikator integriert sein und stellt als solche eine lumineszierende Komponente zur Lagekontrolle vor und während des Eingriffs dar.

Vor dem Eingriff kann die Anatomie, d. h. die Lage, der Verlauf und die Länge der eustachischen Röhre bestimmt werden. Durch das flexible Design des Applikators und eine geeignete Stentauswahl ist eine individuelle Anpassung an die Anatomie des Patienten möglich.

Eine stabile Handhabung des Applikators ist trotz des geringen Durchmessers möglich. Der Applikator ist so stabil ausgeführt, dass zum Beispiel ein Nasensporn nicht das System während des Eingriffs verdrehen würde. Ein Schaft aus Edelstahl mit der besagten Dicke von 1,8 bis 2,3 mm ist ausreichend, um eine hinreichende Festigkeit und Biegesteifigkeit für eine stabile Handhabung zu gewährleisten. Durch einen inneren Arbeitskanal mit einem Durchmesser kleiner als oder gleich 0,8 mm, der sich vorzugsweise über das gesamte System erstreckt, ist es möglich, Führungsdrähte für die Platziersicherheit zu verwenden. Alternativ kann eine lumineszierende Komponente zur Lagekontrolle eingesetzt werden.

In Kombination mit einer formstabilen Schleuse, welche den Stent schützt, d. h. mit einer Kombination aus Außentubus und Innenteil, kann trotz der distalen Krümmung eine reibungsfreie Stentfreisetzung garantiert werden. Insbesondere ist eine reibungsfreie Stentfreisetzung auch dann möglich, wenn sowohl das Innenteil als auch der Außentubus durch plastische Verformung mittels Handkraft in die gewünschte koaxial verlaufende Position gebogen werden.

Der erfindungsgemäße Applikator kommt mit einem Stent aus einem Werkstoff mit superelastischen Materialeigenschaften, nämlich mit einem Stent aus Nitinol zum Einsatz. Im gecrimpten Zustand kann ein solcher, selbstexpandierender Nitinol-Stent einen Durchmesser von 1 bis 2,5 mm haben. Die Wandstärke kann in einem Bereich von 0,09 bis 0,15 mm liegen. Durch die Wandstärke und auch den Durchmesser des Stents können die Radialkräfte des Stents eingestellt werden. Bei zu geringer Kraft wird die Tubendysfunktion nicht geöffnet. Zu viel Kraft führt zu einer klaffenden Tube. Die richtige Einstellung soll die Ventilfunktion der Tuben erhalten, d. h. die Muskulatur unterstützen und das passive Öffnen der Tube bei großen Druckunterschieden erleichtern.

Der verwendete Stent besitzt insbesondere ein geschlossenzelliges Design. Ein solches Design ermöglicht es, den Stent um eine gekrümmte Bahn zu führen, wie sie durch den erfindungsgemäßen Applikator einstellbar ist.

Der Stent ist an die Anatomie einer eustachischen Röhre angepasst. Hierzu besitzt er ein erstes Ende und ein zweites Ende, wobei die Enden unterschiedliche Durchmesser haben. Ein zur Anordnung am Tubenostium ausgebildetes erstes Ende besitzt einen Durchmesser von 4,5 bis 5,5 mm, vorzugsweise einen Durchmesser von 4,8 bis 5,2 mm. In einem besonders bevorzugten Ausführungsbeispiel beträgt der Durchmesser am Tubenostium 5 mm.

Das zur Anordnung am knöchernen Isthmus ausgebildete zweite Ende besitzt einen kleineren Durchmesser und zwar in einem Bereich von 2,5 bis 3,5 mm, insbesondere in einem Bereich von 2,8 bis 3,2 mm. In einem besonders bevorzugten Ausführungsbeispiel beträgt der Durchmesser des zweiten Endes 3 mm.

Der Übergang von dem im Durchmesser größeren Bereich zu dem im Durchmesser kleineren Bereich erfolgt über einen sich im Durchmesser verkleinernden Längenabschnitt. Dieser Längenabschnitt erstreckt sich über 20 bis 40% der Länge des Stents, vorzugsweise über einen Bereich von 25 bis 35%. In einem besonders bevorzugten Ausführungsbeispieles erstreckt sich der Längenabschnitt über eine Länge von 30% der Länge des Stents. Dieser im Durchmesser verkleinernde Längenabschnitt ist insbesondere konisch ausgebildet. Bei einer Länge des Stents von 8 bis 40 mm beträgt die Länge des sich im Durchmesser verkleinernden Längenabschnitts ca. 3 bis 12 mm, vorzugweise 4 bis 9 mm.

Die Erfindung wird nachfolgend anhand von in den Zeichnungen rein schematisch dargestellten Ausführungsbeispielen erläutert. Es zeigen:
- Figur 1: eine schematische Darstellung eines Applikators;
- Figur 2: eine zweite Darstellung eines Applikators mit gebogener Spitze;
- Figur 3: einen Stent zur Verwendung mit dem erfindungsgemäßen Applikator in einer Seitenansicht und
- Figur 4: den Stent der Figur 3 in einer Stirnansicht.

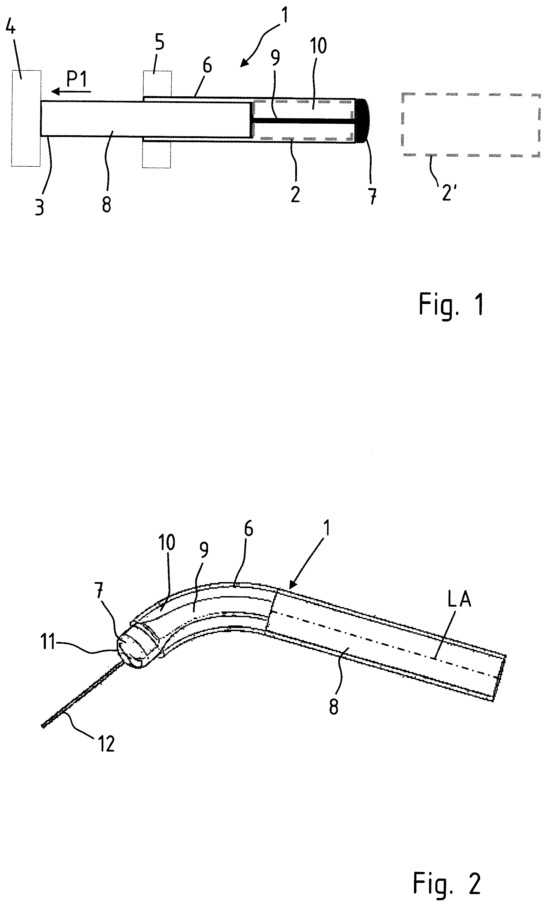

Die Figur 1 zeigt den stark vereinfacht dargestellten Applikator 1 zum Platzieren eines Stents 2 in einer nicht näher dargestellten eustachischen Röhre. Der Applikator 1 besitzt an seinem proximalen Ende 3 einen verbreiterten Anschlag 4 für einen Griff 5, der an einem Außentubus 6 des Applikators 1 befestigt ist. Der Griff 5 kann in Richtung des Pfeils P1 zum Anschlag 4 verschoben werden. Dadurch kann der Stent 2, der innerhalb des Außentubus 6 gehalten ist, am distalen Ende 7 des Applikators freigegeben werden. Der Stent besteht aus Nitinol. Er ist selbstexpandierend. Mit dem Bezugszeichen 2' ist der freigegebene Stent in seiner expandierten Form rein symbolisch dargestellt.

Der Applikator 1 besitzt einen langen schlanken Schaft 8, der verkürzt dargestellt ist. Er kann einen sich über die gesamte Länge erstreckenden Längskanal aufweisen. Im Bereich des distalen Endes 7 befindet sich am Schaft 8 ein im Durchmesser reduzierter Bereich. Hierbei handelt es sich um ein Innenteil 9, das sich in dem in Längsrichtung verlagerbaren Außentubus 6 befindet. Der Durchmesser im Innenteil 9 ist soweit reduziert, dass in einem Ringraum 10 der Stent 2 vor der Freigabe platziert werden kann. Der Ringraum 10 kann auch als Schleuse bezeichnet werden. Das Innenteil 9 und der Außentubus 6 sind mittels Handkraft verformbar.

Das Ausführungsbeispiel der Figur 2 zeigt einen solchen Applikator 1 mit einem verformten Innenteil 9. Die zu der Figur 1 eingeführten Bezugszeichen werden für die Bezeichnung von im Wesentlichen funktionsgleichen Komponenten auch für die Figur 2 beibehalten. Der Schaft 8 ist wie bei dem Ausführungsbeispiel der Figur 1 von dem Außentubus 6 umgeben, der wiederum in Richtung der Längsachse LA des Schaftes 8 verlagerbar ist. In dem Ringraum 10 kann der Stent angeordnet sein. Das distale Ende 7 besitzt eine gerundete Spitze 11.

Dieser Applikator 1 ist mit einem Längskanal versehen, der den Applikator 1 über die gesamte Länge durchsetzt. Innerhalb des Längskanals ist ein Lichtleiter angeordnet. Der Lichtleiter ist mit einer Lichtquelle verbunden. Dadurch kann an der Spitze 11 ein Lichtstrahl 12 austreten. Der verformbare Bereich des Applikators kann um bis zu 70° gebogen werden, bezogen auf die Längsaches LA im nicht gebogenen Längenbereich des Apllikators 1.

Der vordere Bereich des Applikators, d.h. das Innenteil 9 und der dortige Bereich des Außentubus 6 sind bogenförmig gekrümmt. Die Biegung ist mittels Handkraft hergestellt worden. Darüber hinaus ist zu erkennen, dass der Außentubus 6 der Biegung des Innenteils 9 im Wesentlichen koaxial folgt, sodass der Ringraum 10 einen im Wesentlichen konstanten Querschnitt hat.

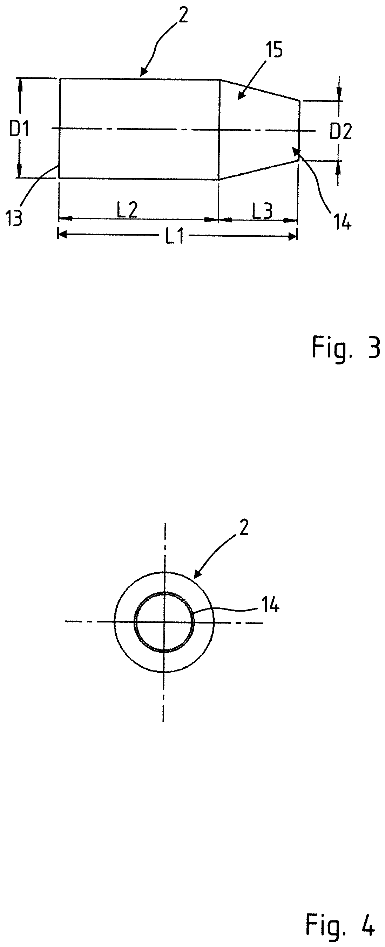

Ein Ausführungsbeispiel eines Stents 2, der bevorzugt mit einem solchen Applikator 1 verwendet wird, ist in der Figur 3 dargestellt. Es besitzt eine Länge L1. Ein zylindrischer Längenabschnitt mit der Länge L2 erstreckt sich über zwei Drittel der Länge L1 des Stents 2. Es schließt sich ein im Durchmesser kleiner werdender Längenabschnitt 15 mit einer Länge L3 an, der sich über ein Drittel der Länge L1 des Stents 2 erstreckt. Der Durchmesser D1 ist in der Bildebene links, d. h. am ersten Ende 13, daher größer als an dem gegenüberliegenden zweiten Ende 14. Der größere Durchmesser D1 beträgt z.B. 5 mm und der kleinere Durchmesser D2 3 mm. Die gesamte Länge L1 des Stents 2 beträgt bei diesem Ausführungsbeispiel 12 mm. Die Figur 4 zeigt den Stent 2 der Figur 3 in einer Stirnansicht auf das zweite, im Durchmesser verjüngte Ende 14.

### Bezugszeichen:

1 - Applikator
2 - Stent
2' - expandierter Stent
3 - proximales Ende von 1
4 - Anschlag
5 - Griff an 6
6 - Außentubus von 1
7 - distales Ende von 1
8 - Schaft von 1
9 - Innenteil von 1
10 - Ringraum von 1
11 - Spitze von 1
12 - Lichtstrahl
13 - erstes Ende von 2
14 - zweites Ende von 2
15 - der im Durchmesser reduzierte Bereich von 2
D1 - Durchmesser von 13
D2 - Durchmesser von 14
LA - Längsachse von 1
L1 - Länge von 2
L2 - Länge
L3 - Länge von 15

## Patentansprüche

1. System, welches einen Applikator (1) zum Platzieren eines Stents (2) in einer eustachischen Röhre und den Stent (2) umfasst, mit folgenden Merkmalen:
a) Der Applikator (1) besitzt ein proximales Ende (3) zur Handhabung des Applikators (1) und ein distales Ende (7) zur Aufnahme eines in einer eustachischen Röhre platzierbaren Stents (2);
b) Das distale Ende (7) weist ein Innenteil (9) und einen Außentubus (6) auf, der das Innenteil (9) im radialen Abstand umgibt und der einen Ringspalt (10) zur Aufnahme des Stents (2) begrenzt, wobei der Stent (2) in dem Ringspalt (10) aus Nitinol besteht und selbst expandierend ist;
**gekennzeichnet durch** folgende Merkmale:
c) Das Innenteil (9) ist zumindest bereichsweise mittels Handkraft plastisch verformbar ausgebildet, zur Anpassung an einen patientenabhängigen Zugangswinkel der eustachischen Röhre;
d) Der Stent (2) weist ein zur Anordnung am Tubenostium ausgebildetes erstes Ende (13) mit einem Durchmesser (D1) von 4,5 bis 5,5 mm auf und ein zur Anordnung am knöchernen Isthmus ausgebildetes zweites Ende (14) mit einem kleineren Durchmesser (D2) als das erste Ende (13), wobei der kleinere Durchmesser (D2) einem Bereich von 2,5 bis 3,5 mm liegt, wobei der Stent (2) benachbart zum zweiten Ende (14) einen sich im Durchmesser verkleinernden Längenabschnitt (15) aufweist, der sich über 20% bis 40% der Länge (L1) des Stents (2) erstreckt.

2. System nach Anspruch 1, **dadurch gekennzeichnet, dass** das verformbare Innenteil (9) eine Länge von 20 bis 40 mm aufweist.

3. System nach Anspruch 1 oder 2, **dadurch gekennzeichnet, dass** das distale Ende (7) des verformbaren Innenteils (9) eine Orientierung aufweist, die bis zu 70° von einer Längsachse (LA) des Applikators (1) abweicht.

4. System nach einem der Ansprüche 1 bis 3, **dadurch gekennzeichnet, dass** der Außentubus (6) zumindest im Bereich des verformbaren Innenteils (9) aus einem plastisch verformbaren Werkstoff besteht.

5. System nach einem der Ansprüche 1 bis 4, **dadurch gekennzeichnet, dass** der Applikator (1) eine gerundete Spitze (11) mit einem Durchmesser von 1,9 bis 2,6 mm aufweist.

6. System nach einem der Ansprüche 1 bis 5 **dadurch gekennzeichnet, dass** ein Schaft (8) des Applikators (1) aus Edelstahl besteht.

7. System nach Anspruch 6, **dadurch gekennzeichnet, dass** der Schaft (8) einen Durchmesser von 1,8 bis 2,3 mm aufweist.

8. System nach einem der Ansprüche 1 bis 7, **dadurch gekennzeichnet, dass** er einen Arbeitskanal aufweist mit einem Durchmesser kleiner oder gleich 0,8 mm.

9. System nach einem der Ansprüche 1 bis 8 **dadurch gekennzeichnet, dass** in dem Innenteil (9) eine bewegliche Lichtleitfaser (12) angeordnet ist zur Lagekontrolle vor dem Freisetzen des Stents (2).

10. System nach einem der Ansprüche 1 bis 9, **dadurch gekennzeichnet, dass** der Stent (2) einen einstellbaren Crimpdurchmesser von 1 bis 2,5 mm aufweist.

11. System nach einem der Ansprüche 1 bis 10, **dadurch gekennzeichnet, dass** der Stent (2) eine Wandstärke in einem Bereich von 0,09 bis 0,15 mm aufweist.

12. System nach einem der Ansprüche 1 bis 11, **dadurch gekennzeichnet, dass** der Stent (2) ein geschlossenzelliges Design aufweist, wobei er um eine gekrümmte Bahn führbar ist.

13. System nach einen der Ansprüche 1 bis 12, **dadurch gekennzeichnet, dass** der sich im Durchmesser verkleinernde Längenabschnitt (15) konisch ausgebildet ist.

14. System nach einem der Ansprüche 1 bis 13, **dadurch gekennzeichnet, dass** er eine Länge (L1) von 8 bis 40 mm aufweist.

## Claims

1. A system, comprising an applicator (1) for placing a stent (2) in a Eustachian tube and the stent (2), having the following features:
a) the applicator (1) has a proximal end (3) for handling the applicator (1) and a distal end (7) for receiving a stent (2) that can be placed in a Eustachian tube;
b) the distal end (7) has an inner part (9) and an outer tube (6) which surrounds the inner part (9) at a radial distance and which delimits an annular gap (10) for receiving the stent (2), wherein the stent (2) in the annular gap (10) consists of nitinol and is self-expanding;
**characterised by** the following features:
c) the inner part (9) is designed to be plastically deformable by means of a manual force, at least in regions, in order to adapt to a patient-dependent access angle of the Eustachian tube;
d) the stent (2) has a first end (13), which is designed for arrangement on the tubal ostium, with a diameter (D1) of 4.5 to 5.5 mm and a second end (14), which is designed for arrangement on the bony isthmus, with a smaller diameter (D2) than the first end (13), wherein the smaller diameter (D2) is in a range of 2.5 to 3.5 mm, wherein the stent (2) has, adjacent to the second end (14), a length section (15) which decreases in diameter and extends over 20% to 40% of the length (L1) of the stent (2).

2. The system according to claim 1, **characterised in that** the deformable inner part (9) has a length of 20 to 40 mm.

3. The system according to claim 1 or 2, **characterised in that** the distal end (7) of the deformable inner part (9) has an orientation which deviates by up to 70° from a longitudinal axis (LA) of the applicator (1).

4. The system according to any one of claims 1 to 3, **characterised in that** the outer tube (6) consists of a plastically deformable material at least in the region of the deformable inner part (9).

5. The system according to any one of claims 1 to 4, **characterised in that** the applicator (1) has a rounded tip (11) with a diameter of 1.9 to 2.6 mm.

6. The system according to any one of claims 1 to 5, **characterised in that** a shaft (8) of the applicator (1) consists of stainless steel.

7. The system according to claim 6, **characterised in that** the shaft (8) has a diameter of 1.8 to 2.3 mm.

8. The system according to any one of claims 1 to 7, **characterised in that** it has a working channel with a diameter less than or equal to 0.8 mm.

9. The system according to any one of claims 1 to 8, **characterised in that** a movable optical fibre (12) is arranged in the inner part (9) for position control before the stent (2) is released.

10. The system according to any one of claims 1 to 9, **characterised in that** the stent (2) has an adjustable crimp diameter of 1 to 2.5 mm.

11. The system according to any one of claims 1 to 10, **characterised in that** the stent (2) has a wall thickness in a range of 0.09 to 0.15 mm.

12. The system according to any one of claims 1 to 11, **characterised in that** the stent (2) has a closed-cell design, wherein it can be guided around a curved path.

13. The system according to any one of claims 1 to 12, **characterised in that** the length section (15) which decreases in diameter is designed conically.

14. The system according to any one of claims 1 to 13, **characterised in that** it has a length (L1) of 8 to 40 mm.

## Revendications

1. Système, comprenant un applicateur (1) pour la mise en place d'un stent (2) dans une trompe d'Eustache, et comprenant le stent (2), présentant les caractéristiques suivantes :
a) l'applicateur (1) comprend une extrémité proximale (3) pour la manipulation de l'applicateur (1) et une extrémité distale (7) pour la réception d'un stent (2), apte à être placé dans une trompe d'Eustache ;
b) l'extrémité distale (7) présente une partie intérieure (9) et un tube extérieur (6) qui entoure la partie intérieure (9) à distance radiale et qui délimite une fente annulaire (10) pour recevoir le stent (2), dans lequel le stent (2) est constitué de nitinol dans la fente annulaire (10) et est auto-expansible ;
**caractérisé par** les caractéristiques suivantes :
c) la partie intérieure (9) est réalisée de manière à être au moins partiellement déformable plastiquement par force manuelle, afin de s'adapter à un angle d'accès de la trompe d'Eustache, qui varie selon les patients ;
d) le stent (2) présente une première extrémité (13) présentant un diamètre (D1) compris entre 4,5 mm et 5,5 mm, destinée à être disposée sur l'ostium du tube auditif, et une deuxième extrémité (14) présentant un diamètre (D2) plus petit que la première extrémité (13), destinée à être disposée sur l'isthme osseux, le diamètre (D2) plus petit étant compris entre 2,5 mm et 3,5 mm, dans lequel le stent (2) présente, à proximité de la deuxième extrémité (14), une section longitudinale (15) dont le diamètre diminue et qui s'étend sur 20 % à 40 % de la longueur (L1) du stent (2).

2. Système selon la revendication 1, **caractérisé en ce que** la partie intérieure (9) déformable présente une longueur de 20 à 40 mm.

3. Système selon la revendication 1 ou la revendication 2, **caractérisé en ce que** l'extrémité distale (7) de la partie intérieure (9) déformable présente une orientation qui s'écarte jusqu'à 70° d'un axe longitudinal (LA) de l'applicateur (1).

4. Système selon l'une quelconque des revendications 1 à 3, **caractérisé en ce que** le tube extérieur (6) est constitué d'un matériau déformable plastiquement au moins dans la zone de la partie intérieure (9) déformable.

5. Système selon l'une quelconque des revendications 1 à 4, **caractérisé en ce que** l'applicateur (1) présente une pointe arrondie (11) avec un diamètre de 1,9 à 2,6 mm.

6. Système selon l'une quelconque des revendications 1 à 5, **caractérisé en ce qu'**une tige (8) de l'applicateur (1) est constituée d'acier inoxydable.

7. Système selon la revendication 6, **caractérisé en ce que** la tige (8) présente un diamètre de 1,8 à 2,3 mm.

8. Système selon l'une quelconque des revendications 1 à 7, **caractérisé en ce qu'**il présente un canal de travail avec un diamètre inférieur ou égal à 0,8 mm.

9. Système selon l'une quelconque des revendications 1 à 8, **caractérisé en ce qu'**une fibre optique mobile (12) est disposée dans la partie intérieure (9) pour contrôler la position avant la libération du stent (2).

10. Système selon l'une quelconque des revendications 1 à 9, **caractérisé en ce que** le stent (2) présente un diamètre de sertissage réglable de 1 à 2,5 mm.

11. Système selon l'une quelconque des revendications 1 à 10, **caractérisé en ce que** le stent (2) présente une épaisseur de paroi dans une plage de 0,09 à 0,15 mm.

12. Système selon l'une quelconque des revendications 1 à 11, **caractérisé en ce que** le stent (2) présente une conception à cellules fermées, dans lequel il est orientable autour d'une trajectoire courbe.

13. Système selon l'une quelconque des revendications 1 à 12, **caractérisé en ce que** la section longitudinale (15) de diamètre décroissant est réalisée de manière conique.

14. Système selon l'une quelconque des revendications 1 à 13, **caractérisé en ce qu'**il présente une longueur (L1) de 8 à 40 mm.
